# EUROPEAN PATENT APPLICATION

(11) **EP 3 449 992 A1**
(43) Date of publication of application: **06.03.2019**
(21) Application number: 17189289.6
(22) Date of filing: 04.09.2017
(51) Int. Cl.: B01D 15/18, A61K 31/352, C07D 311/80

(54) **RECOVERY OF ACIDIC CANNABINOIDS FROM PLANT MATERIAL**

(71) Applicant: Bionorica Ethics GmbH, 92318 Neumarkt (DE)
(72) Inventor: POPP, Johannes Richard, 96253 Untersiemau (DE); BONN, Günther, 6170 Zirl (AT); STUPPNER, Hermann, 6091 Götzens (AT); SKALTSOUNIS, Alexios Leandros, 157 71 Zographou (GR); PETRAKIS, Eleftherios A., 157 71 Zographou (GR); ANGELIS, Apostolis, 157 71 Zographou (GR); HALABALAKI, Maria, 157 71 Zographou (GR)
(74) Representative: Simandi, Claus

(57) **Abstract**

The invention relates to cannabinoids and their isolation and purification and to obtaining them by means of centrifugal partition chromatography and pH-zone refining.

## Description

The invention relates to cannabinoids and concerns the recovery, isolation, and purification thereof by means of Centrifugal Partition Chromatography (CPC).

CPC is used for obtaining and enriching plant content substances from botanical extracts on the analytical, semi-preparative and preparative scale. CPC is a liquid-liquid chromatography method that uses a generally two-phase solvent system.It permits nearly loss-free separation of highly complex mixtures of substances from crude extracts. Manufacturers of such centrifugal partition chromatographs for performing CPC are e.g. Kromaton S.a.r.l (Annonay, FR) and Armen Instrument S.a.s. (Saint-Avé, FR).

A two basically immiscible liquid solvent phases are used in the CPC method. The upper (lighter) phase and the lower (heavier) phase may alternatively be selected to as the stationary phase. CPC provides a rotor carrying a plurality of serially connected separation chambers. In the separation process, the rotor is rotating rapidly (e.g. up to 2,500 rpm). Because of this, firstly, the liquid stationary phase is retained in the separation chambers due to the centrifugal force, and, secondly, the separation of the two phases is accelerated due to the centrifugal force. This allows greater flow speeds and consequently enables the throughput of large quantities of substances at high flow-rates in a short period of time so that this separation technology also has preparative application.

The mobile phase migrates through the separation chambers and thus through the liquid stationary phase. In the so-called "ascending mode" the mobile phase is the lighter phase and ascends through the heavier stationary phase. In the so-called "descending mode" the mobile phase is the heavier phase and descends in the lighter stationary phase.

The separation coefficient K of the desired substance between the two phases should be in the range between 0.7 and 4.5. If K is lower, the substance elutes too rapidly, so that no separation can take place. If K is higher, the retention time for rapid purification of large quantities of a botanical extract becomes too long.

The prior art describes that cannabinoids may be obtained from cannabis extract by means of CO₂ extraction (DE10051427C1). Nevertheless, the cannabinoids, such as e.g. dronabinol (Δ9-THC) and cannabidiol (CBD) are never obtained with absolute purity.

In fresh plant material of cannabis, most cannabinoids are present in the form of carboxylic acid, known as acidic cannabinoids, such as the tetrahydrocannabinolic acids (THCA), in particular Δ9-THCA, cannabigerolic acids (CBGA), and cannabidiolic acid (CBDA). The free phenolic forms are known as neutral cannabinoids, such as the tetrahydrocannabinoles (THC), in particular Δ9-THC (dronabinol), cannabigerols (CBG), and cannabidiols (CBD).

Hazekamp, A. et al., Preparative isolation of cannabinoids from Cannabis sativa by centrifugal partition chromatography, J. Liq. Chrom. Rel. Technol. 2004, 27(15),2421-2439, describe the preparative recovery and large-scale isolation of acidic cannabinoids (THCA, CBGA, CBDA) by means of CPC in a two-phase system based on hexane/methanol/water. The maximum purity attained is 93.1%. Even from drug-type cannabis female flower tops only low yields of 17% are attained.

The object is therefore to provide an improved method for separating and/or purifying cannabinoids, specifically acidic cannabinoids from cannabis plants (incl. hemp) or botanical extracts therefrom. The method shall eliminate the identified drawbacks and in particular is able to provide a higher yield and/or purity of acidic cannabinoids, especially CBDA and/or THCA, in a large-scale preparation.

This technical problem is fully solved by the provision of a novel method for the preparation, i.e. separating and/or purifying, of cannabinoids, specifically in the form of acidic cannabinoids, from cannabis plants or botanical extracts as set forth in the claims.

More specifically, a first aspect of the invention provides a method for separating and/or purifying acidic cannabinoids from cannabis plant extract, the method comprising the initial step (a) of setting-up, starting or preparing a biphasic liquid-liquid centrifugal partition chromatography (CPC)for pH-zone refining, characterized in that the liquid stationary upper phase is the lighter phase and is an acidified non-polar phase, comprising at least one organic non-polar solvent and at least one acidic component, and in that the liquid mobile lower phase is the heavier phase and is and is a neutral or basic polar phase, comprising at least one polar solvent and at least one basic component.

In a preferred embodiment, the acidic upper phase has a pH from 1 to 3, more preferred pH 2. The acidity is preferably obtained by addition of one or more acidic compound, preferably selected from strong organic acids dissolvable in non-polar solvents, in particular trifluoracetic acid (TFA).

In a preferred embodiment, the neutral to basic lower phase has a pH from 7 to 11, more preferred pH 10. The basic pH is preferably obtained by addition of one or more of basic compound, preferably selected from strong organic bases dissolvable in polar solvents, in particular triethylamine (Et3N).

In a preferred embodiment, the acidic upper phase comprises n-hexane/ethyl acetate and the lower phase comprises ethanol/water. Preferably volume ratio of n-hexane/ethyl acetate/ethanol/water is 7-9/1-3/4-6/4-6 (v/v), more specifically 8/2/5/5 (v/v). Although this is the preferred biphasic system of solvents, other biphasic systems of similar properties can be selected (see below).

In the substantial working step (b) of the method the extract is subjected to said prepared CPC and fractions of the extract are consecutively eluted therefrom. In step (c) the acidic cannabinoids collected from one or more of the fractions, in particular from fractions identified to contain the acidic cannabinoid(s) of interest, for example, in preceding runs.

In a preferred embodiment, step b) may include one or more or all further sub-step(s) which are described in the following their sequential order:
b1) injecting the extract diluted in the acidified upper phase into the CPC immediately followed by injecting neutral to basic lower phase to elute a first set of fractions of the extract in descending mode at a first flow rate of the lower phase;
   and optionally:
b2) injecting the lower phase to elute a second set of fractions of the extract in descending mode at a second flow rate of the lower phase, which is 1.2 to 2.0-fold, preferably 1.5-fold, higher than the first flow rate;
   and optionally:
b3) injecting the upper phase to obtain a third set of fractions of the extract in ascending mode at a third flow rate, which preferably is 1.5 to 3-fold, more preferred 2-fold, higher than the first flow rate.

In a most preferred embodiment, particularly step c) is followed by the following step(s):
d) purifiying one or more or all fractions obtained or selected in the preceeding steps by, preferably repeated, (biphasic) liquid-liquid extraction (LLE), wherein the polar water phase has a pH from 0.5 to 2, preferably pH 1. In particular, the non-polar organic phase comprises n-hexane/ethyl acetate, at a preferred volume ration of 1/1, and the polar water phase comprises water and a strong (inorganic) acid, preferably hydrochloric acid (HCl).

After purification, the organic fraction or, if repeated, fractions may be collected and pooled and the organic solvent is evaporated to obtain the final product: a highly pure acidic cannabinoid or a composition thereof.

The use of the method of the invention for the preparation of acidic cannabinoids from cannabis plant material provides acidic cannabinoids at a recovery rate of pure CBDA (>95% purity) of greater than 40% or at a recovery rate of CBDA (>85% purity) of greater than 85%, respectively.

Thus, in a second aspect the present invention provides an acidic cannabinoid composition, obtainable by the method of the invention, characterized in that the purity of CBDA is greater than 95%.

The present invention also provides cannabidiolic acid, obtainable by the method of the invention, having a purity greater than 95%.

The inventive method advantageously demonstrates lower eluent consumption in addition to no deposits on a stationary phase, so that it is not necessary to regenerate the stationary phase or to prepare or remove interfering components. Product recovery is nearly quantitative, since stationary and mobile phases may simply be exchanged. In addition, the stationary phase is completely renewed during each run and may be purified or renewed in a simple manner using distillation. The purity of the isolated cannabinoids is often so high that no additional chromatographic purification steps are needed. It is also simple to execute the method on the industrial scale. The yield of cannabinoids is often sharply higher compared to the prior art, as shall be explained in greater detail in the following. The number of process stages required may generally be significantly reduced compared to alternative techniques.

It is particularly advantageous that cannabinoids may be obtained from any desired cannabis plants or their extracts. This includes cannabis extracts from cannabis plants (*Cannabis sativa, Cannabis indica, Cannabis ruderalis*)*,* such as hemp, industrial hemp, pharmaceutical hemp, fiber hemp, etc.

"Cannabis extract" in the context of this invention means any processed extract from a cannabis or hemp plant that includes cannabinoids. The extract may be a primary extract or may be a partly processed extract. The manufacture of cannabis extracts is adequately described in the prior art. Suitable cannabis plants or (fiber) hemp plants are those such as pharmaceutical hemp or fiber hemp.

Examples according to the invention of further solvent systems include (stationary phase/mobile phase): n-heptane/acetonitrile (1/1) (without t-butyl methyl ether), n-heptane/ethylacetate/acetonitrile, n-heptane/ethylacetate/t-butyl methyl ether/acetonitrile, n-heptane/ethylacetate/methanol/water, n-heptane/methanol/water, n-heptane/ethanol/water, n-heptane/ acetone/water, n-heptane/methanol/acetonitrile, n-heptane/ ethanol/acetonitrile, n-heptane/acetone/acetonitrile, n-heptane/ chloroform/acetonitrile, n-heptane/chloroform/ methanol, n-heptane/methanol, n-heptane/ethanol/methanol, n-heptane/n-butanol/acetonitrile, n-heptane/2-propanol/water, n-heptane/n-propanol/water, n-heptane/2-propanol/acetonitrile, n-heptane/n-propanol/acetonitrile, n-heptane/dichloromethane/acetonitrile, n-heptane/dichloromethane/methanol, n-heptane/tetrahydrofuran/acetonitrile, n-heptane/benzotrifluoride/acetonitrile, Cyclohexane/methanol/water, Cyclohexane/methanol/acetonitrile, Cyclohexane/t-butyl methyl ether/water, Cyclohexane/acetonitrile/water, Isooctane/methanol, Isooctane/methanol/water, Isooctane/ethyl acetate/methanol/water. Acetonitrile, possibly with 1-15% t-butyl methyl ether (TBME) added, is particularly advantageous.

When using a 1000 mL CPC rotor, it is also advantageously possible to purify up to 9 g extract per run.

In the context of this invention, especially the following substances shall be understood to be cannabinoids:
*Cannabigerol-type (CBG) :* Cannabigerol ((E)-CBG-C₅), cannabigerol monomethyl ether ((E)-CBGM-C₅ A), cannabinerolic acid A ((Z)-CBGA-C₅ A), cannabigerovarin ((E)-CBGV-C₃), cannabigerolic acid A ((E)-CBGA-C₅ A), cannabigerolic acid A monomethyl ether ((E)-CBGAM-C₅ A), Cannabigerovaric acid A ((E)-CBGVA-C₃ A);
*Cannabichromene-type (CBC) :* Cannabichromene (CBC-C₅), cannabichromene acid A (CBCA-C₅ A), cannabichromevarin (CBCV-C₃), cannabichromevarinic acid A (CBCVA-C3 A);
*Cannabidiol-type (CBD):* Cannabidiol (CBD-C₅), cannabidiol monomethyl ether (CBDM-C₅), cannabidiol-C4 (CBD-C₄), cannabidivarin (CBDV-C₃), cannabidiorcol (CBD-C₁), cannabidiolic acid (CBDA-C₅), cannabidivarinic acid (CBDVA-C₃);
*Cannabinodiol-type (CBND) :* Cannabinodiol (CBND-C₅), cannabinodivarin (CBND-C₃);
*Tetrahydrocannabinol-type (THC):* Δ9-tetrahydrocannabinol (Δ9-THC-C₅), Δ9-tetrahydrocannabinol-C4 (Δ9-THC-C₄), Δ9-tetrahydrocannabivarin (Δ9-THCV-C₃), Δ9-tetrahydrocannabiorcol (Δ9-THCO-C₁), Δ9-tetrahydrocannabinolic acid (Δ9-THCA-C₅ A), Δ9-tetrahydrocannabinolic acid B (Δ9-THCA-C₅ B), Δ9-tetrahydrocannabinolic acid-C4 (Δ9-THCA-C₄ A and/or B), Δ9-tetrahydrocannabivarinic acid A (Δ9-THCVA-C₃ A), Δ9-tetrahydrocannabiorcolic acid(Δ9-THCOA-C₁ A and/or B), (-)-Δ8-trans-(6aR,10aR)-Δ8-tetrahydrocannabinol (Δ8-THC-C₅),(-)-Δ8-trans-(6aR,10aR)-tetrahydrocannabinolic acid A (Δ8-THCA-C₅ A); (-)-(6aS,10aR)-Δ9-tetrahydrocannabinol ((-)-cis-Δ9-THC-C₅);
*Cannabinol-type (CBN) :* Cannabinol CBN-C₅, cannabinol-C4 (CBN-C₄), cannabivarin (CBN-C₃), cannabinol-C2 (CBN-C₂), cannabiorcol (CBN-C₁), cannabinolic acid A (CBNA-C₅ A), cannabinol methylether (CBNM-C₅)
*Cannabitriol-type (CBT) :* (-)-(9R,10R)-trans-cannabitriol ((-)-trans-CBT-C₅), (+)-(9S,10S)-cannabitriol ((+)-trans-CBT-C₅), (±)-(9R,10S/9S,10R)-cannabitriol ((±)-cis-CBT-C₅), (-)-(9R,10R)-trans[10-0-ethyl-cannabitriol] ((-)-trans-CBT-OEt-C₅), (±)-(9R,10R/9S,10S)-cannabitriol-C3 ((±)-trans-CBT-C₃), 8, 9-dihydroxy-Δ6a(10a) tetrahydrocannabinol (8,9-Di-OH-CBT-C₅), cannabidiolic acid A (CBDA-C₅ 9-OH-CBT-C5 ester), (-)-(6aR,9S,10S,10aR)-9,10-dihydroxy-hexahydrocannabinol, cannabiripsol cannabiripsol-C5, (-)-6a,7,10a-trihydroxy-A9-tetrahydrocannabinol ((-)-cannabitetrol), 10-oxo-Δ6a(10a) tetrahydrocannabinol (OTHC);
*Cannabielsoin-type (CBE) :* (5aS,6S,9R,9aR)- C₅-cannabielsoin (CBE-C₅), (5aS,6S,9R,9aR)-C₃-cannabielsoin (CBE-C₃), (5aS,6S,9R,9aR)-cannabielsoic acid A (CBEA-C₅ A),
(5aS,6S,9R,9aR)-cannabielsoic acid B (CBEA-C₅ B), (5aS,6S,9R,9aR)-C3-cannabielsoic acid B (CBEA-C₃ B), cannabiglendol-C3 (OH-iso-HHCV-C₃), dehydrocannabifuran (DCBF-C₅), cannabifuran (CBF-C₅);
*Isocannabinoids:* (-)-Δ7-trans-(1R,3R,6R)-isotetrahydrocannabinol, (±)-Δ7-1,2-cis-(1R,3R,6S/1S,3S,6R)-isotetrahydrocannabivarin, (-)-Δ7-trans-(1R,3R,6R)-isotetrahydrocannabivarin;
*Cannabicyclol-type (CBL) :* (±)-(1aS,3aR,8bR,8cR)-cannabicyclol (CBL-C₅), (±)-(1aS,3aR,8bR,8cR)-cannabicyclolic acid A (CBLA-C₅ A), (±)-(1aS,3aR,8bR,8cR)-cannabicyclovarin (CBLV-C₃);
*Cannabicitran-type (CBT) :* Cannabicitran (CBT-C₅) ;
*Cannabichromanone-type (CBCN):* Cannabichromanone (CBCN-C₅), cannabichromanone-C3 (CBCN-C₃), cannabicoumaronone (CBCON-C₅).

In the context of the present invention, the term "liquid-liquid centrifugal partition chromatography step" shall be construed especially to mean chromatography which proceeds as follows:

A specific quantity of a substance mixture is conducted, with a liquid mobile phase, through a phase that is kept stationary, wherein the latter is kept stationary using centrifugal force. In another preferred embodiment, the liquid-liquid centrifugal partition chromatography may be performed continuously. To this end, the two phases are conducted in the counter-current and continuous separation occurs, instead of a temporally offset (discontinuous) output (see, e.g., Yin, Lianhong; Li, Yingnan; Lu, Binan; Jia, Yujie; Peng, Jinyong, Trends in Counter-Current Chromatography: Applications to Natural Products Purification Separation and Purification Reviews (2010), 39(1-2), 33-62).

Corresponding to its interactions with the stationary phase, which interactions differ in strength, the substances exit continuously or discontinuously and may be separated. However, while the stationary phase in liquid chromatography comprises a packed solid bed in a column, in liquid-liquid centrifugal partition chromatography there is a second, non-miscible liquid phase that is kept stationary by suitable devices, such as e.g. a rotor, using centrifugal force, in particular by means of a corresponding centrifugal partition chromatograph (see above).

In another preferred embodiment, at least one preparative column (solid phase, such as e.g. silica gel) may be performed, upstream or downstream.

The following examples and figures are intended solely for illustrating the invention, without limiting the invention to these examples.

The figures show:
Fig. 1 TLC chromatogram of the combined fractions (CS-01 to 11) obtained by CPC along with the SFE-CO2 hemp extract; mobile phase DCM/MeOH 95:5 (v/v).
Fig.2 TLC chromatogram indicating CBDA purity in fraction CS-03 before and after liquid-liquid extraction (LLE); mobile phase DCM/MeOH 95:5 (v/v).

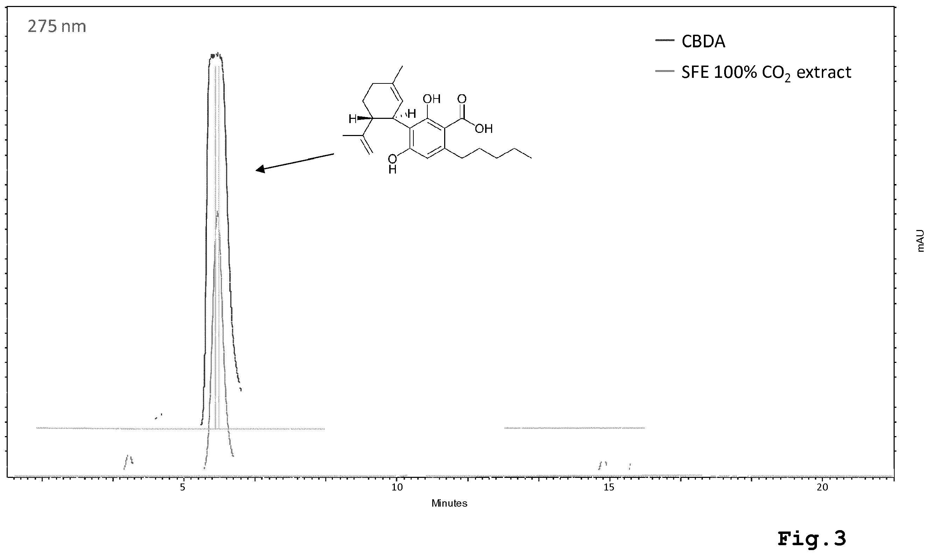

Fig.3 HPLC-UV chromatograms of CBDA (>95%) and SFE-CO2 hemp extract; detection at 275 nm.

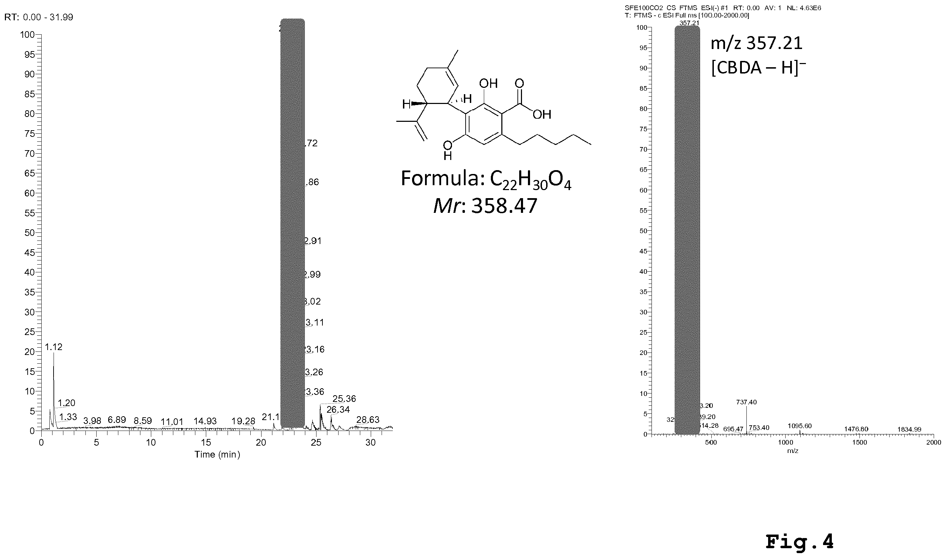

Fig.4 LC-HRMS and ESI(-)-HRMS analysis of CS-03 fraction after LLE (CBDA>95%).

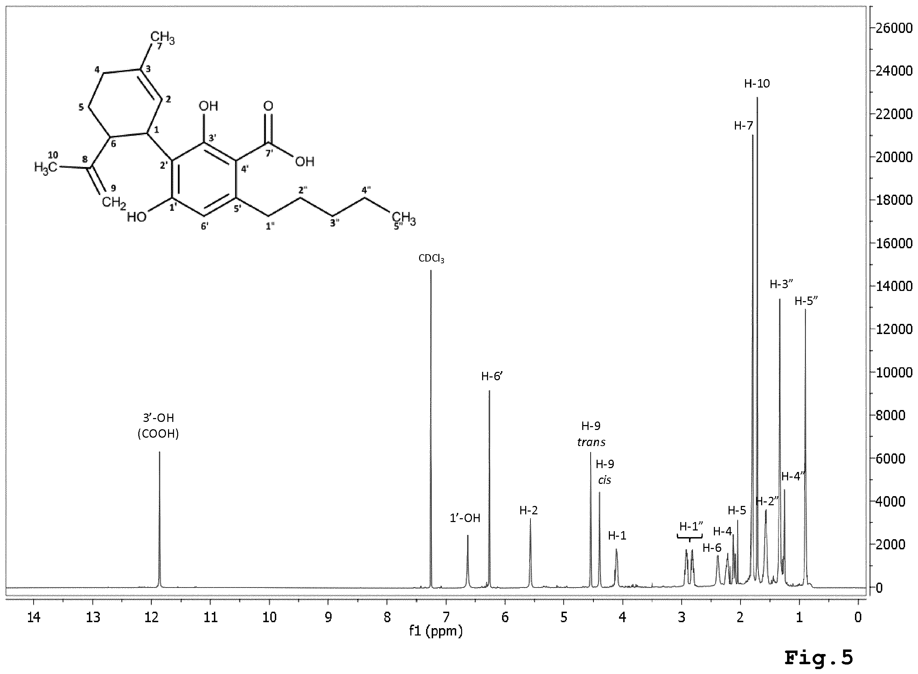

Fig.5 1H-NMR spectrum of isolated CBDA (>95%), in CDCl3.

### Example: pH-Zone centrifugal chromatography of cannabinoids

### 1. Materials and methods

### 1.1. Reagents and materials

Aerial parts of Cannabis sativa L. var. 'Fedora 17' (Cannabaceae) were collected by a local producer in Ipato (central Greece), during the flowering period in October 2016. After the collection, the plant material was air-dried at 25 °C and ground in a mill until a fine powder was obtained. It was stored at room temperature in the absence of light. CPC separations were performed using solvents of analytical grade, i.e. ethyl acetate (EtOAc), ethanol (EtOH), and n-hexane (n-Hex), purchased from Carlo Erba Reactifs SDS (Val de Reuil, France). Water, acetonitrile (ACN), and formic acid of HPLC grade (Carlo-Erba) were used for HPLC analysis. Methanol (MeOH), dichloromethane (DCM), hydrochloric acid, and sulphuric acid (>95%) were obtained from Fisher Scientific (Leicestershire, UK), while vanillin (98%), trifluoroacetic acid (TFA) and triethylamine (TEA) were purchased from Sigma-Aldrich (Steinheim, Germany).

### 1.2. Supercritical Fluid Extraction (SFE)

In this work, the supercritical fluid extraction of C. sativa was performed using a pilot-scale apparatus (SFE 1-2 No. 4218, SEPAREX, Champigneulles, France). It consists of a CO2 tank, a liquid CO2 pump (that can deliver up to 10 kg/h), two directly connected extraction vessels (i.e. 1 & 2 L), three separators (200 mL capacity each), a co-solvent pump, and a cooling system. The extraction was carried out within a stainless steel basket placed in the 2 L tubular extractor. The pressure in the extraction vessels was kept constantly at 15 MPa, while the pressure in the separators compartment was held at 4 MPa. The extraction and separation temperature were 35 °C and 30 °C, respectively. The extraction was static with continuous recycling of the CO2 gas at a flow rate of 5 kg/h. The extract was partially collected every 40 min and the whole SFE procedure lasted approximately 30 h. Finally, the extract was submitted to lyophilisation and the total yield was estimated as % (w/w) of the dried plant material.

### 1.3. Centrifugal Partition Chromatography (CPC)

The fractionation of the lyophilised SFE extract from C. sativa was performed by CPC using a pH-zone refining method. CPC experiments employed the use of a Kromaton FCPC® instrument equipped with a rotor (column) of 1000mL, a Laboratory Alliance® pump and a DAD detector controlled by Gravity® 6.2 software. Three wavelengths were chosen to detect eluted compounds; 220, 275 and 324 nm.

The extract was injected into the system through a 50mL loop and eluted fractions were collected with a Buechi B-684 fraction collector. CPC fractionation was performed with pH-zone refining method using the biphasic system of n-hexane/ethyl acetate/ethanol/water 8:2:5:5 (v/v/v/v).

After pre-equilibration of the biphasic system, the two phases were separately collected. The organic (upper) phase, containing n-hexane and ethyl acetate, was adjusted to pH 2 by adding trifluoroacetic acid and was used as the stationary phase. The water (lower) phase was adjusted to pH 10 by adding triethylamine and was used as the mobile phase.

Initially, the column was filled with the stationary phase at a flow rate of 25 mL/min and a constant rotation speed of 200 rpm, in descending mode. Then, 9 g of extract diluted in 40 mL of acidified upper phase and 5 mL of neutral lower phase were injected.

The rotation speed was increased to 650 rpm and the pH-zone refining fractionation was started by pumping the mobile phase in descending mode. The flow rate was set at 10 mL/min for the first 11 fractions, followed by an increase to 15 mL/min to collect 42 more fractions (elution step).

The final step of the experiment comprised the extrusion of the column content by pumping the organic phase (20 mL/min) in ascending mode and the same rotation speed, resulting in the collection of 45 fractions.

All collected fractions (n = 98, 30 mL) were analysed by normal-phase TLC (see below) and those presenting similar chemical composition were pooled to finally obtain 11 combined fractions (CS-01 to 11). Fractions of interest were further analysed by NMR, HPLC, MS, and LC-MS to identify the structure and estimate the purity of the isolated compounds.

### 1.4. Liquid-liquid Extraction (LLE)

The fractions of interest were submitted to purification from triethylamine employing LLE, with n-hexane/ethyl acetate 1:1 (v/v) (solvent A) and a water solution of hydrochloric acid at pH 1 (solvent B) within a separatory funnel.

The organic (non-polar) fractions collected after 4 replicates of the LLE procedure were merged and evaporated to dryness using a rotary evaporator (Buchi Rotavapor R-200) at 45 °C.

### 1.5. Analysis of the fractions obtained

### 1.5.1 Thin-Layer Chromatography (TLC)

TLC analysis of extracts and fractions was performed on Silica gel 60 F254 TLC aluminium plates of 0.1 mm thickness (Merck, Germany). Spots were visualized at 254 and 366 nm as well as by spraying with a 1:1 mixture of 5% (v/v) H2SO4 in MeOH and 10% (v/v) vanillin in MeOH on the dried plates, followed by heating until maximum visualization of spots. Solvent systems consisting of DCM/MeOH 98:2 and 95:5 (v/v) were used for the analysis.

### 1.5.2 High-performance Liquid Chromatography (HPLC)

The HPLC analysis of extracts and fractions was carried out using a Thermo Finnigan HPLC SpectraSystem equipped with a P4000 binary pump, an AS3000 autosampler, a column thermostat and a UV6000LP photodiode array detector (Thermo Finnigan, Germany). ChromQuest 5.0 software was used for the operation of the system and data processing. Samples were injected into an Ascentis C18 3 µm, 150 mm × 2.1 mm column (Supelco, Sigma-Aldrich, USA) and the compounds eluted using a gradient mode of 0.1% formic acid in water (solvent A) and 0.1% formic acid in acetonitrile (solvent B). Separation was achieved under the gradient conditions suggested by Giese et al. (2015) [1] with slight modifications, that is: 66% B for 9 min, followed by a linear gradient to 95% B over 4.5 min; isocratic for 1 min and then from 95 to 66% B in 4.5 min, followed by isocratic conditioning at 66% B for 6 min. Total run time was 25 min and UV spectra were recorded from 200 to 400 nm. The PDA was set at 220, 275 and 324 nm for all compounds. The column temperature was 25 °C, the injection volume was 5 µL and the flow rate was 0.45 mL/min.

### 1.5.4 Liquid Chromatography - Mass Spectrometry (LC-MS)

LC-MS analysis was conducted using an Accela High Speed LC System equipped with a PDA detector and connected to an LTQ-Orbitrap XL hybrid mass spectrometer, using an ESI ionisation probe, in the negative mode (Thermo Scientific, Germany). MS parameters: source voltage: 2.70 kV, source current 100 µA °C; capillary temp.: 300 °C, capillary voltage: -40 V, °C; drying gas: 10 mL/min; nebulizer: 40 psi; full scan mode: m/z 100-1500.

### 1.5.5 Gas Chromatography - Mass Spectrometry (GC-MS)

For GC-MS analysis, C. sativa SFE extract was diluted in DCM at a concentration of 1 mg/mL and an aliquot of 1 µL was injected in splitless mode on a Finnigan Trace GC Ultra 2000 (Thermo Electron Corporation, USA) apparatus, equipped with a Finnigan Trace DSQ mass selective detector (EI mode) and an AI 3000 autosampler. Xcalibur 2.2 software (Thermo Electron Corporation, USA) was used for the operation of the system and data processing. The injector temperature was 220 °C, the detector temperature was 250 °C and the carrier gas (helium) flow was 1 mL/min. The capillary column used was a 30 m-length DB-5 column and the analysis was carried out according to Ref. [2], using a temperature programme starting from 100 °C and increasing to 280 °C at a rate of 10 °C/min. The final temperature remained stable for 12 min. The overall run time was 30 min.

### 1.5.6 Nuclear Magnetic Resonance

NMR spectra were acquired at 298 K by means of a Bruker (Bruker Biospin, Rheinstetten, Germany) Avance III 600 spectrometer, equipped with a 5 mm broadband ATM probe with a z-gradient operating at 600.11 MHz, using chloroform (99.8 atom % D, Euriso-Top, France) as the solvent. Experiments were performed in automation mode, using a Bruker BACS-60 sample changer operated by Bruker IconNMR. The chemical shifts of the compounds were referenced to the residual solvent signals (δ H 7.26 and δ C 77.0 for α-H and α-C, respectively). Data acquisition and processing was performed with TopSpin 2.1 software (Bruker, Germany). All 1D (1H) and 2D NMR (i.e. COSY, HSQC, HMBC) data were acquired using default Bruker pulse experiments with standard acquisition parameters.

### 2. Results

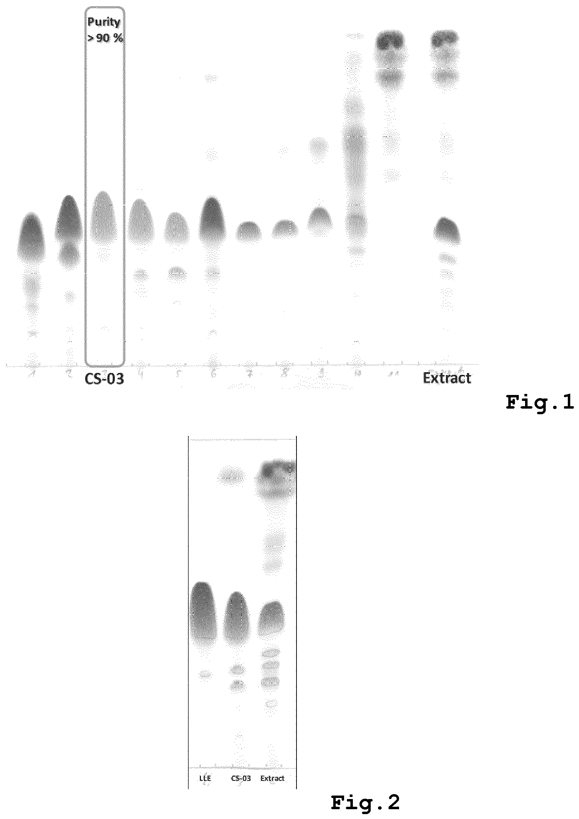

The CPC fractionation using the suggested pH-zone refining method resulted to 98 fractions, which were finally merged into 11 combined fractions (CS-01 to 11). As shown in Fig. 1, CBDA has been collected in fractions CS-02 to CS-06. Among them, fractions CS-03 to CS-05 appeared to be the most promising for the recovery of CBDA. Fraction CS-03 accounts for 13.2% (w/w) of the whole SFE-CO2 hemp extract (Table 1), providing a CBDA purity grade more than 90% according to HPLC-UV analysis.

Thus, CS-03 was submitted to purification from triethylamine employing LLE with n-hexane/ethyl acetate 1:1 (v/v) (solvent A) and a water solution of hydrochloric acid at pH 1 (solvent B) within a separatory funnel.

The organic (non-polar) fractions collected after 4 replicates of the LLE procedure were merged and evaporated to dryness, affording 91% (w/w) recovery of pure CBDA (purity grade >95%, HPLC).

The yield in CBDA (>95%) obtained after CPC and a simple LLE step is considered to be 12.0% (w/w) (Table 1). Accordingly, fractions CS-04 and CS-05 accounts for 4.8% and 8.7% of the total SFE-CO2 hemp extract, with a CBDA purity grade more than 90% and 85%, respectively (Table 1).

Considering that the CBDA content of SFE-CO2 hemp extract is approximately 30% (w/w), this methodology enables a recovery greater than 40% of pure CBDA (>95%, HPLC) and more than 85% for CBDA having a purity > 85% (HPLC).

**Table 1: CBDA yield (% w/w) in three combined fractions, as obtained by pH-zone CPC and optional subsequent LLE.**

| Fraction | Before LLE | | After LLE | | |
|---|---|---|---|---|---|
| | Weight (g) | Yield (% w/w) | Weight (g) | Yield (% w/w) | Purity (HPLC) |
| CS-03 | 1.19 | 13.2 | 1.08 | 12.0 | >95% |
| CS-04 | 0.48 | 5.3 | 0.44 | 4.8 | >90% |
| CS-05 | 0.86 | 9.5 | 0.78 | 8.7 | >85% |
| Estimated CBDA content | | | | 25.5 | |
| Total SFE-CO2 extract | 9.01 | | | | |

## Claims

1. A method for separating and/or purifying acidic cannabinoids from cannabis plant extract, comprising the step(s) of:
a) preparing a biphasic liquid-liquid centrifugal partition chromatography (CPC), **characterized in that**
the liquid stationary upper phase is the lighter phase and is an acidified non-polar phase, comprising at least one organic non-polar solvent and at least one acidic component,
the liquid mobile lower phase is the heavier phase and is and is a neutral or basic polar phase, comprising at least one polar solvent and at least one basic component;
b) subjecting the extract to CPC and eluting fractions of the extract; and
c) collecting acidic cannabinoids from one or more of the fractions.

2. The method of claim 1, wherein the acidic upper phase has a pH from 1 to 3

3. The method of claim 1 or 2, wherein the lower phase has a pH from 7 to 11.

4. The method of any one of the preceding claims, wherein the acidic upper phase comprises n-hexane/ethyl acetate and the lower phase comprises ethanol/water.

5. The method of claim 4, wherein the a volume ratio of n-hexane/ethyl acetate/ethanol/water at 7-9/1-3/4-6/4-6, specifically at 8/2/5/5 (v/v).

6. The method of any one of the preceding claims, step b) further comprising the step(s):
b1) injecting the extract diluted in the acidified upper phase into the CPC immediately followed by injecting neutral to basic lower phase to elute a first set of fractions of the extract in descending mode at a first flow rate of the lower phase.

7. The method of claim 6, step b) further comprising the step (s) :
b2) injecting the lower phase to elute a second set of fractions of the extract in descending mode at a second flow rate of the lower phase, which is 1.2 to 2.0-fold higher than the first flow rate.

8. The method of claim 6 or 7, step b) further comprising the step(s):
b3) injecting the upper phase to obtain a third set of fractions of the extract in ascending mode at a third flow rate.

9. The method of any one of the preceding claims, further comprising the step(s):
d) purifiying the fractions obtained in step c) by biphasic liquid-liquid extraction (LLE), wherein the polar water phase has a pH from 0.5 to 2, preferably pH 1.

10. The method of claim 9, wherein in step d) the non-polar organic phase comprises n-hexane/ethyl acetate and the polar water phase comprises water and a strong inorganic acid.

11. Acidic cannabioid composition, obtainable by the method of any one of claims 1 to 10, **characterized in that** the purity of CBDA is greater than 95%.

12. Cannabidiolic acid, obtainable by the method of any one of claims 1 to 10, wherein the purity is greater than 95%.

13. The use of the method of any one of claims 1 to 10 for the preparation of acidic cannabinoids from cannabis plant material at a recovery rate of pure CBDA (>95% purity) of greater than 40% or at a recovery rate of CBDA (>85% purity) of greater than 85%.
